# EUROPÄISCHE PATENTANMELDUNG

(11) **EP 1 488 785 A1**
(43) Veröffentlichungstag der Anmeldung: **22.12.2004**
(21) Anmeldenummer: 03013790.5
(22) Anmeldetag: 18.06.2003
(51) Int. Cl.: A61K 9/107, A61K 31/565, A61K 31/57

(54) **Ölemulsion zur postnatalen Hormonsubstitution**

(71) Anmelder: B. BRAUN MELSUNGEN AG, 34212 Melsungen (DE)
(72) Erfinder: Brömer, Sonja, Dr., 34212 Melsungen (DE); Nehne, Jörg, Dr., 34202 Guxhagen (DE); Pohlandt, Frank, Prof. Dr. med., 89081 Ulm (DE)
(74) Vertreter: Weber, Thomas, Dr.

(57) **Zusammenfassung**

Die Erfindung betrifft ein Verfahren zur Herstellung hormonhaltiger Ölemulsionen (Lipidemulsionen), eine isotone Ölemulsion erhältlich nach diesem Verfahren sowie die Verwendung der erfindungsgemäßen Emulsion zur Herstellung eines Medikaments zur intravenösen Verabreichung, insbesondere zur postnatalen Hormonsubstitution bei Frühgeborenen und zur Behandlung von neurologischen Schäden nach Schlaganfällen.

## Beschreibung

Die Erfindung betrifft ein Verfahren zur Herstellung hormonhaltiger Ölemulsionen (Lipidemulsionen), eine isotone Ölemulsion erhältlich nach diesem Verfahren sowie die Verwendung der erfindungsgemäßen Emulsion zur Herstellung eines Medikaments zur intravenösen Verabreichung, insbesondere zur postnatalen Hormonsubstitution bei Frühgeborenen und zur Behandlung von neurologischen Schäden nach Schlaganfällen.

### Technischer Hintergrund der Erfindung

Während der Schwangerschaft steigen die Plasmakonzentrationen von 17-β-Östradiol (ein Östrogen) und Progesteron (ein Gestagen) bis um das 100fache an. Diese Mehrsynthese von Östrogen und Progesteron dient u.a. der Aufrechterhaltung der Schwangerschaft. Aus Nabelschnurblutuntersuchungen zu verschiedenen Zeitpunkten während der Schwangerschaft geht hervor, dass auch der Fetus diesen hohen Plasmakonzentrationen ausgesetzt ist. Es bestehen deutliche Hinweise dafür, dass die fetale Entwicklung verschiedener Organe, wie z.B. die Lunge, der Knochen und das Gehirn, von Östrogen und Progesteron abhängig ist. Termingerecht erfolgt nach 40 Schwangerschaftswochen die Geburt, nach der es sowohl beim Neugeborenen als auch bei der Mutter zu einem rapiden Abfall von Östradiol und Progesteron kommt. Dieser Konzentrationsabfall der Hormone ist vermutlich für die bei Frauen nach der Geburt häufig auftretende Depression mitverantwortlich.

In der Bundesrepublik Deutschland sind 6 - 7 % aller Neugeborenen Frühgeborene, d.h. der Geburtstermin liegt vor der vollendeten 37. Schwangerschaftswoche. Der rapide Abfall der Plasmakonzentrationen von Östradiol und Progesteron findet also zu einem früheren Zeitpunkt während der fetalen Entwicklung statt, was Folgen für die noch unausgereiften Organe haben könnte. Eine unreife Lungenfunktion bei extrem kleinen Frühgeborenen (unter 1000 g Geburtsgewicht) ist auch heute noch eine wesentliche Ursache der Neugeborenensterblichkeit. Folgeuntersuchung ehemaliger Frühgeborener zeigen auch, dass die neurologische Entwicklung langfristig beeinträchtigt ist.

Das Frühgeborene von einer Aufrechterhaltung der hohen Plasmakonzentrationen von Östradiol und Progesteron profitieren könnten, zeigen Untersuchungen an 30 Frühgeborenen von *Trotter et al.* an der Kinderklinik der Universität Ulm (veröffentlicht in *J Clin Endocrinol Metab* **84,** 4531 - 4355 (1999)). Frühgeborenen wurde eine verdünnte Lipidemulsion kontinuierlich intravenös verabreicht, die die Hormone 17-β-Östradiol und Progesteron in Mengen enthielt, die ausreichten, um die im Mutterleib vorzufindenden Plasmakonzentrationen aufrechtzuerhalten. Dabei zeigte sich, dass die Frühgeborenen eine im Median bessere Mineralisation des Knochens aufwiesen. Außerdem konnte beobachtet werden, dass bei hormonbehandelten Frühgeborenen im Alter von 28 Tagen weniger häufig die zusätzliche Gabe von Sauerstoff nötig war. Diese Beobachtung kann auf eine bessere Lungenreifung zurückgeführt werden. Die intravenöse Gabe der Sexualhormone erfolgte durchschnittlich über 3 Wochen hinweg.

Eine Nachuntersuchung, der in der Pilotstudie behandelten Frühgeborenen im korrigierten Alter von 15 Monaten lässt darüber hinaus einen positiven Einfluss auf die neurologische Entwicklung erkennen (veröffentlich in *J Clin Endocrinol Metab* **86,** 601 - 603 (2001).

Eine transepidermale Behandlung mit den entsprechenden Hormonen ist prinzipiell möglich, kann aber aus entwicklungsspezifischen Gründen erst 2 - 3 Wochen postnatal begonnen werden.

Die bisher vorliegenden Ergebnisse lassen einen positiven Einfluss der Hormonbehandlung auf die Zielkriterien Lungen-Reifung und -Entwicklung, neurologische Entwicklung und Knochenmineralisierung, also insgesamt eine Verbesserung der Reifung der Frühgeborenen, erkennen. Zusätzlich dient die parenteral verabreichte Lipidemulsion auch reinen Ernährungszwecken, indem sie dem frühgeborenen Organismus, bei dem sich eine enterale Nahrungszufuhr oft schwierig gestaltet, Öl in einer intravenös verträglichen Darreichungsform zuführt.

Bei der zuvor beschriebenen Studie wurden die Hormone Östrogen und Progesteron in einer alkoholischen Lösung der von der Firma Pharmacia & Upjohn hergestellten und unter dem Handelsnamen Intralipid® vertriebenen Lipidemulsion zugesetzt und verabreicht. Die Ölphase von Intralipid® besteht zu 100 % Sojabohnenöl in Form langkettiger Triglyceride.

Genauere chemische Analysen zeigen, dass beim Zumischen der Sexualhormone zu Intralipid® erhebliche Unsicherheiten bestehen. Beispielsweise ist unklar, ob die Mischung der Steroide in Intralipid® überhaupt physikalisch stabil ist. Experimente mit radioaktiv markierten Hormonen zeigten jedoch, dass ein erheblicher Teil der applizierten Hormone an der Oberfläche der Infusionssysteme und Zuleitungen absorbiert wird. Daraus resultieren notwendigerweise Probleme und Unsicherheiten hinsichtlich der Verfügbarkeit der intravenös applizierten Hormone beim Säugling.

Außerdem hat sich im Verlauf der zuvor beschriebenen Studien gezeigt, dass die Verabreichung von relativ großen Mengen an hormonhaltiger Ölemulsion nur zu vergleichbar geringen Hormonspiegeln im Blut des Frühgeborenen führte. Zur Erzielung der gewünschten Serumspiegel mussten den Frühgeborenen daher bislang relativ hohe Mengen an Ölemulsionen verabreicht werden. Diese führten zum einen zu einer unerwünschten Öl- und Flüssigkeitsbelastung des Frühgeborenen. Im Übrigen stellt die Gabe alkoholischer Zusammensetzungen an Frühchen keine bevorzugte Behandlungsmethode dar.

Das der Erfindung zugrunde liegende Problem ist daher die Bereitstellung von parenteral verabreichbaren Ölemulsionen, die bei minimaler Öl- und Volumenzufuhr zu einer möglichst hohen Anreicherung der Hormone (Verfügbarkeit) im Blut des Frühgeborenen führen.

Überraschenderweise wurde gefunden, dass die parenterale Verabreichung einer Östradiol- und Progesteron-haltigen Ölemulsion, bei deren Herstellung die Hormone vor dem Emulgieren in der Ölphase gelöst werden, zu deutlich höheren Serumkonzentrationen führt, als die Verabreichung von Emulsionen, denen die Hormone direkt zugesetzt werden.

### Figurenbeschreibung

**Figur 1** beschreibt den Östradiol-Plasmaspiegel bei den mit den verschiedenen Emulsionen behandelten Frühgeborenen zu den einzelnen Messzeitpunkten (Tag 1, 3, 7, 14).
**Figur 2** beschreibt den Progesteron-Plasmaspiegel bei den mit den verschiedenen Emulsionen behandelten Frühgeborenen zu den einzelnen Messzeitpunkten (Tag 1, 3, 7, 14).

### Beschreibung der Erfindung

Die vorliegende Erfindung betrifft ein Verfahren zur Herstellung isotoner östrogenund gestagenhaltiger Ölemulsionen zur parenteralen, vorzugsweise intravenösen Verabreichung umfassend die Schritte
(A) Lösen der Hormone in einer Ölphase und
(B) Emulgieren der Öl- in der Wasserphase
in Gegenwart eines Emulgators.

Eine weitere Ausführungsform der Erfindung betrifft eine hormonhaltige isotone Ölemulsion zur intravenösen Applikation, die nach dem obigen Verfahren erhältlich ist. In einer bevorzugten Ausgestaltung liegen in der Emulsion Gestagen und Östrogen im Verhältnis von 2:1 bis 200:1 vor.

Die erfindungsgemäßen O/W-Emulsionen sind für die parenterale, insbesondere intravenöse Verabreichung geeignet. Dementsprechend betrifft eine dritte Ausführungsform der Erfindung die Verwendung der obigen isotonen Ölemulsion zur intravenösen Verabreichung, insbesondere zur postnatalen Hormonsubstitution bei Frühgeborenen.

Die Begriffe Öl und Lipid sind im Zusammenhang mit der vorliegenden Erfindung von identischer Bedeutung und werden daher austauschbar verwendet. Unter dieser Gruppe sind insbesondere Triglyceride, Partialglyceride und Fettsäurereste und deren Mischungen zu verstehen.

Die erfindungsgemäßen Ölemulsionen sind insbesondere geeignet für Verfahren zur Herstellung von Medikamenten zur parenteralen Applikation von Östrogenen und Gestagenen, vorzugsweise zur postnatalen Hormonsubstitution bei Frühgeborenen.

Weitere Ausführungsformen der Erfindung ergeben sich aus den abhängigen Ansprüchen.

Das Ziel der postnatalen Substitution von Gestagen und Östrogen ist die Aufrechterhaltung der intrauterin vorkommenden Plasmakonzentrationen (Plasmakonzentration *in utero*). Um nach der Geburt möglichst wenig von den intrauterinen Plasmakonzentrationen abzuweichen, ist deren schnellstmögliches Erreichen nach Beginn der Substitution wünschenswert.

Die Hormonsubstitution ist nicht auf menschliche Frühgeborene beschränkt, sondern kann auch auf Tiere, vorzugsweise Säugetiere angewendet werden.

Die erfindungsgemäßen Ölemulsionen weisen eine deutlich bessere Verfügbarkeit der darin enthaltenen Hormone gegenüber derjenigen einer Ölemulsion des Standes der Technik auf.

Die erfindungsgemäße Ölemulsion führt, verglichen mit Emulsionen des Standes der Technik, zu einem schnelleren Anstieg der Hormonkonzentration und zu einer höheren Östrogen Endkonzentration bei geringerer Volumen- und Ölzufuhr.

Zusätzlich weisen die erfindungsgemäßen Ölemulsionen eine verbesserte Stabilität verglichen mit den alkoholischen Hormonlösungen des Standes der Technik auf.

Der entscheidende Schritt des erfindungsgemäßen Verfahrens zur Herstellung der Ölemulsionen ist das Lösen der Hormone Östradiol und Progesteron in der Ölphase vor dem Emulgieren der Ölphase mit der Wasserphase. Grundsätzlich werden beide Hormone in der Ölphase gelöst. In einer weiteren erfindungsgemäßen Ausgestaltung wird nur eines der Hormone, vorzugsweise ein Östrogen, in der Ölphase gelöst, während das Gestagen der Wasserphase und/oder der fertigen Emulsion zugesetzt wird.

Die im Zusammenhang mit der vorliegenden Erfindung verwendeten Hormone sind solche, die auch intrauterin vorkommen. Dabei unterscheidet man insbesondere die Follikelhormone (Östrogene) und die Hormone des Gelbkörpers (Gestagene).

Die im Zusammenhang mit der vorliegenden Erfindung bedeutsamen Follikelhormone sind Östron, 17-β-Östradiol und Östriol und deren Derivate. Aufgrund seiner hohen östrogenen Wirksamkeit ist 17-β-Östradiol für die vorliegende Erfindung von besonderer Bedeutung.

Die im Zusammenhang mit der vorliegenden Erfindung verwendeten Gelbkörperhormone sind Pregnelonon, Progesteron, Medroxyprogesteron und deren pharmazeutisch akzeptablen Derivate, wobei Progesteron im Zusammenhang mit der vorliegenden Erfindung bevorzugt verwendet wird.

Eine Ausführungsform der Erfindung betrifft die Kombination von Östron mit Pregnelonon und/oder Progesteron, eine weitere die Kombination von Östriol mit Pregnelonon und/oder Progesteron. Eine alternative, besonders bevorzugte Ausführungsform betrifft die Kombination von 17-β-Östradiol und/oder Pregnelonon und/oder Progesteron, insbesondere mit Progesteron. Darüber hinaus können jedoch auch mehr als zwei Hormone miteinander kombiniert werden.

Die erfindungsgemäßen Lipidemulsionen weisen Hormonkonzentrationen auf, die bei entsprechender Anwendung zu Konzentrationen im Frühgeborenen führen, wie sie im Mutterleib zu erwarten gewesen wären. Folglich umfasst die erfindungsgemäße Lipidemulsion zwischen 0,005 und 0,5 Gew.-%, vorzugsweise zwischen 0,01 und 0,2 Gew.-%, besonders bevorzugt zwischen 0,05 und 0,1 Gew.-% wenigstens eines Östrogens und zwischen 0,05 und 5 Gew.-%, vorzugsweise zwischen 0,1 und 2 Gew.-% , besonders bevorzugt zwischen 0,5 und 1 Gew.-% wenigstens eines Gestagens, bezogen auf die Gesamtzusammensetzung (Stammemulsion).

Das Verhältnis von Gestagen zu Östrogen in der Emulsion beträgt 2:1 bis 200:1, vorzugsweise 5:1 bis 50:1, besonders bevorzugt 10:1 bis 20:1.

Zur besseren Dosierung der Ölemulsionen können die Stammemulsionen bei Bedarf mit einer entsprechenden Menge, vorzugsweise mit der bis zu vierfachen Menge Wassers verdünnt werden.

Die erfindungsgemäßen Lipidemulsionen werden vorzugsweise aus Ölen pflanzlichen Ursprungs (z.B. Safloröl oder Sojabohnenöl) und/oder MCT und/oder Ölen tierischen Ursprungs hergestellt. Sie können daher Pflanzenöl und/oder mittelkettige Triglyceride (MCT) und/oder Öle marinen Ursprungs (z.B. Fischöle) enthalten. Derartige Lipidemulsionen sind dem Fachmann aus dem Stand der Technik bekannt.

Pflanzenöle und insbesondere die Öle der Sojabohne und der Saflordistel sind durch einen hohen Anteil an mehrfach ungesättigten Fettsäuren aus der ω-6-Reihe gekennzeichnet (überwiegend Linolsäure, 18:2 ω-6), während ihr Gehalt an ω-3-Fettsäuren (praktisch ausschließlich als α-Linolensäure, 18:3 ω-3) gering ist.

Mittelkettige Triglyceride (MCT) haben eine Kettenlänge von C₆ bis C₁₄, besonders bevorzugt ist dabei eine Kettenlänge von C₈ bis C₁₀.

Die mit den Ölemulsionen verabreichten mittelkettigen Triglyceride (MCT) dienen vorwiegend als Energiequelle. Mittelkettige Triglyceride enthalten überhaupt keine ungesättigten Fettsäuren und somit weder ω-6- noch ω-3-Fettsäuren.

Die aus Kaltwasserfischen gewonnenen Fischöle sind durch einen hohen Anteil an mehrfach ungesättigten Fettsäuren gekennzeichnet (in der Hauptsache Eikosapentaensäure, EPA, 20:5 ω-3 und Docosahexaensäure, DHA, 22:6 ω-3), während ihr Gehalt an ω-6-Fettsäuren gering ist. Geeignete Fischöle sind beispielsweise solche, wie sie technisch in bedeutendem Umfang aus Kaltwasserfischen gewonnen werden. Fischöle enthalten im allgemeinen Triglyceride von Fettsäuren mit 12 bis 22 Kohlenstoffatomen. Besonders bevorzugt werden hochgereinigte Fischölkonzentrate, die beispielsweise aus Sardinenöl, Lachsöl, Heringöl und/oder Makrelenöl gewonnen werden.

Eine erfindungsgemäße Ausführungsform betrifft daher eine Ölemulsion auf Basis von Pflanzenöl und/oder MCT. Diese Emulsion kann wahlweise Fischöl aufweisen.

Der Anteil an Pflanzenöl in der erfindungsgemäßen Öl-Zusammensetzung beträgt dabei wenigstens 50 bis 100 Gew.-%, vorzugsweise 70 bis 100 Gew.-%, besonders bevorzugt 90 bis 100 Gew.-% bezogen auf die Öl-Zusammensetzung.

Die vorteilhafte Wirkung von ungesättigten Fettsäuren, insbesondere jener der ω-3-Reihe, ist dem Fachmann bekannt und wurde beispielsweise in EP-A-0311091 und DE-A-19648566 beschrieben. Auch im Zusammenhang mit der vorliegenden Erfindung, kann die Verwendung von Ölen, die reich an ungesättigten Fettsäuren sind, vorteilhaft sein.

Der Gesamtölgehalt der Stammemulsion beträgt zwischen 1 Gew.-% und 30 Gew.-%, vorzugsweise zwischen 10 Gew.-% und 20 Gew.-%, bezogen auf die wässrige Ölemulsion.

Die isotone Ölemulsion kann neben destilliertem Wasser noch die üblichen Hilfsund/oder Zusatzstoffe wie Emulgatoren, Emulgierhilfsstoffe (Co-Emulgatoren), Stabilisatoren, Antioxidantien und Isotonisierungszusätze enthalten.

Als Emulgatoren werden physiologisch verträgliche Emulgatoren, wie Phospholipide tierischen oder pflanzlichen Ursprungs verwendet. Besonders bevorzugt sind gereinigte Lecithine, insbesondere Eilecithin oder Fraktionen daraus oder die entsprechenden Phosphatide. Der Emulgatorgehalt beträgt 0,6 Gew.-% bis 1,5 Gew.-%, vorzugsweise 1,2 Gew.-% bezogen auf die Gesamtemulsion.

Als Emulgierhilfsstoffe können weiterhin Alkalisalze langkettiger C₁₆ bis C₂₀ Fettsäuren verwendet werden. Besonders bevorzugt sind deren Natriumsalze. Die Emulgierhilfsstoffe werden in einer Konzentration von 0,005 Gew.-% bis 0,1 Gew.-%, vorzugsweise von 0,01 Gew.-% bis 0,5 Gew.-%, bezogen auf die Gesamtemulsion eingesetzt.

Zur Stabilisierung und Isotonisierung kann die erfindungsgemäße Emulsion von 1,0 Gew.-% bis 8 Gew.-%, vorzugsweise 2,0 Gew.-% bis 6,0 Gew.-%, besonders bevorzugt 2,2 Gew.-% bis 2,6 Gew.-% eines Stabilisierungs- oder Isotonisierungszusatzes, beispielsweise eines mehrwertigen Alkohols, enthalten. Bevorzugt ist in diesem Zusammenhang Glycerin, Glucose, oder Xylit, wobei Glycerin besonders bevorzugt ist.

Die erfindungsgemäße Ölemulsion kann als Antioxidanz und somit zum Schutz vor Peroxidbildung Tocopherole oder physiologisch unbedenkliche Tocopheroloester, z.B. alpha-Tocopherolacetat, in einer Menge von 10 bis 1000 mg, vorzugsweise 25 bis 200 mg, bezogen auf 100 g Öl, enthalten.

Es versteht sich dabei von selbst, dass keine Hilfsstoffe verwendet werden, die unerwünschte Nebenwirkungen oder Unverträglichkeiten zur Folge haben. Insbesondere Fructose und Sorbit stehen in dem Verdacht Unverträglichkeiten hervorzurufen und sind daher im Zusammenhang mit der vorliegenden Erfindung ungeeignet. Des weiteren enthalten die erfindungsgemäßen Ölemulsionen keine Konservierungsmittel, wie z.B. Benzylalkohol.

Bei den erfindungsgemäßen Ölemulsionen handelt es sich stets um Öl-in-Wasser-(O/W)-Emulsionen, bei denen die äußere, zusammenhängende Phase aus destilliertem, für parenterale Zwecke geeignetem Wasser besteht.

Die Ölemulsion weist vorteilhafterweise einen pH-Wert von 6,0 bis 9,0, vorzugsweise von 6,5 bis 8,5 auf.

Die erfindungsgemäßen isotonen wässrigen Lipidemulsionen können nach bekannten Verfahren hergestellt werden. Üblicherweise geht man hierzu so vor, dass man zunächst die Öle, den Emulgator und andere Hilfs- und Zusatzstoffe miteinander mischt und anschließend unter Dispergieren mit Wasser auffüllt. Das Wasser kann gegebenenfalls noch weitere wasserlösliche Komponenten (z.B. Glycerin) enthalten.

Die so erhaltene Emulsion weist noch Tröpfchengrößen von etwa 10 µm auf. Die mittlere Tröpfchengröße der Emulsion muss durch weitere Homogenisierung z.B. durch Verwendung eines Hochdruckhomogenisators weiter reduziert werden. Bevorzugt für die parenterale, insbesondere für die intravenöse Anwendung sind Tröpfchengrößen mit einem mittleren Teilchendurchmesser von 0,5 µm bis 150 nm, besonders bevorzugt 1 µm bis 100 nm. Die Lösungen sollen darüber hinaus sterilisierbar und wenigstens über 18 Monate hinweg stabil lagerfähig sein.

Neben der Verwendung der erfindungsgemäßen isotonen Ölemulsion zur parenteralen, insbesondere intravenösen Applikation von Östrogen und Gestagen und, in einer alternativen Ausführungsform, zur Herstellung eines Medikaments zu diesem Zweck, hat sich gezeigt, dass die erfindungsgemäßen Ölemulsionen das Absterben von Nervenzellen im Gehirn von Menschen und Tieren, vorzugsweise Säugetieren reduzieren und daher auch zur Herstellung von Medikamenten zur Behandlung neurologischer Schäden nach Schlaganfällen (Apoplex) eingesetzt werden können. Die erfindungsgemäßen Ölemulsionen können auch prophylaktisch angewendet werden. In solchen Fällen ist eine orale Verabreichung der Ölemulsionen zu bevorzugen.

Die erfindungsgemäße isotone Ölemulsion kann in Verfahren zur Hormonsubstitution bei Frühgeborenen als auch zur Behandlung neurologischer Schäden nach Schlaganfällen angewandt werden.

Die Erfindung wird durch nachfolgende Beispiele erläutert, ohne sie jedoch auf diese zu beschränken.

### Beispiele

### Herstellung einer hormonhaltigen Ölemulsion:

Die Hormone Estradiol und Progesteron werden in dem auf ca. 70 °C erwärmten Öl unter Inertisierung mit Stickstoff gelöst, gegebenenfalls unter Zusatz von Tocopherol (= Lösung I). Der Emulgator (= Phospholipide aus Ei) wird mittels eines Ultra-Terrax in einer wässrigen Glycerinlösung dispergiert (= Komponente II). Die Lösung I wird unter Verwendung eines Ultra-Terrax zur Komponente II gegeben. Der pH-Wert der daraus resultierenden o/w-Emulsion wird durch Zugabe von Natriumoleat auf ca. 8,5 eingestellt. Anschließend wird in einem Hochdruckhomogenisator bei mindestens 400 kg/cm² homogenisiert.

Nach Abfüllung in Glasampullen geeigneter Qualität wird nach bekannten Verfahren hitzesterilisiert. Es resultiert eine sterile, stabile o/w-Emulsion mit Lipidtröpfchen mit einer mittleren Öltröpfchengröße von kleiner 0,5 µm mit einer Lagerungsstabilität von mindestens 18 Monaten.

**TABELLE 1**

| | **Herstellungsbeispiel** | **1** | **2** | **3** | **4** |
|---|---|---|---|---|---|
| I. | Estradiol-Hemihydrat | 0,66 g | 0,66 g | 0,60 g | 0,60 g |
| | Progesteron | 6,00 g | 6,00 g | 6,00 g | 6,00 g |
| | Mittelkettige Triglyceride | - | 100 g | 100 g | 200 g |
| | Gereinigtes Sojaöl | 200 g | 100 g | 80 g | - |
| | Hochgereinigtes Fischöl | - | - | 20 g | - |
| | α-Tocopherol | - | - | 200 mg | - |
| II. | Gereinigte Phospolipide aus: | 12 g Ei | 12 g Ei | 12 g Ei | 12 g Ei |
| | Glycerin | 25 g | 25 g | 25 g | 25 g |
| | *Aqua ad injectabilia* | *ad* 1 Liter | *ad* 1 Liter | *ad* 1 Liter | *ad* 1 Liter |
| | Natriumoleat | 0,3 g | 0,3 g | 0,25 g | 0,25 g |

### Anwendung einer hormonhaltigen Ölemulsion

Die Patientengruppe, die mit einer Hormonemulsion des Standes der Technik behandelt wurde, bestand aus 12 Patienten (Frühgeborene < 29 Schwangerschaftswochen, Geburtsgewicht unter 1000 g). Diese wurde mit einer Hormonemulsion bestehend aus 20 Gew.-% Intralipid® (Firma Pharmacia & Upjohn, Deutschland) verdünnt mit isotonischer Kochsalzlösung auf 5 % Ölgehalt, die mit einer ethanolischen Lösung von 0,15 mg/ml kristallinem 17 β-Östradiol und 1,4 mg/mL Progesteron versetzt wurde, behandelt.

Die initiale Zufuhr wurde mit 15 ml/kg/Tag kontinuierlich i.v. begonnen. Sowohl der Hormongehalt der Emulsion als auch die Flüssigkeitsmenge konnten variiert werden. Damit kam es zu Flüssigkeitszufuhren bis maximal 25,8 ml/kg/Tag, Median 18,8).

In einer derzeit laufenden randomisierten Doppelblindstudie zum Einfluss einer Hormonsubstitution auf das Zielkriterium zusätzlicher Sauerstoffbedarf im Alter von 28 Tagen wurden bereits 78 Frühgeborene rekrutiert. In dieser Studie wurde die erfindungsgemäße Hormonemulsion (gemäß Herstellungsbeispiel 1, mit *aqua ad injectabilia* auf einen Ölgehalt von 5 % verdünnt) bzw. eine Placebo (hormonfreie Emulsion, Ölgehalt 5 %) eingesetzt. Von 52 Patienten liegen bereits Ergebnisse zu erreichten Plasmaspiegeln von Östradiol und Progesteron vor. Ohne die Verblindung aufzulösen, kann anhand der Spiegel abgeleitet werden, dass 25 Patienten mit hormonhaltiger Lösung behandelt wurden (Verumgruppe). Es wurden allen Patienten 15 ml/kg/Tag der erfindungsgemäßen Emulsion kontinuierlich i.v. verabreicht. Daraus lässt sich eine mittlere Hormonzufuhr von 2,47 mg/kg/Tag Östradiol und 22,5 mg/kg/Tag Progesteron ableiten.

Die Plasmakonzentrationen von Östradiol und Progesteron am 1. (24 Stunden), 3., 7., und 14. Lebenstag unter laufender Hormonzufuhr wurden in beiden Gruppen bestimmt. Die Ergebnisse dieser Untersuchungen sind in der folgenden Tabelle dargestellt:

**TABELLE 2**

| **Hormon** | **Emulsion des Standes der Technik** | | **Erfindungsgemäße Emulsion** | | |
|---|---|---|---|---|---|
| | **Zufuhr (mg/kg/d)** | **Plasmakonz. (ng/ml)**^{***)**} | **Zufuhr (mg/kg/d)** | **Plasmakonz. (ng/ml)**^{***)**} | **P** |
| Östradiol | 2,23 | 2,622 | 2,48 | 4,270 | 0,00038 |
| Progesteron | 20,23 | 286 | 22,50 | 292 | 0,153 |

| | | | | | |
|---|---|---|---|---|---|
| ^{*)} median | | | | | |

Tabelle 1 zeigt die mediane Zufuhr von Östradiol und Progesteron bis zum 14. Lebenstag für die Frühgeborenen beider Gruppen. Beide Emulsionen führten zu einer ähnlich hohen Hormonzufuhr. Während die erfindungsgemäße Emulsion konstant mit 15 ml/kg/Tag zugeführt wurde, wurde die Emulsion des Standes der Technik im Median mit 18,8 ml/kg/Tag (Min-Max: 11,4 bis 25,8 ml/kg/Tag) verabreicht.

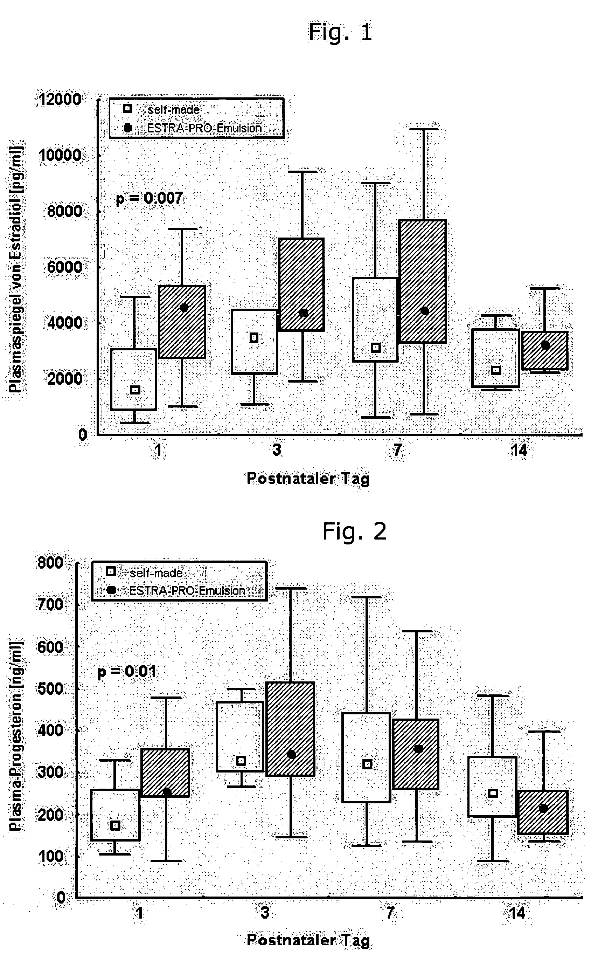

Mit der erfindungsgemäßen Emulsion konnten signifikant höhere Plasmakonzentrationen für Östradiol bei den Frühgeborenen erreicht werden (p = 0,00038). Bei den Progesteron-Plasmakonzentrationen zeigte sich jedoch kein signifikanter Unterschied, betrachtet man den gesamten Zeitraum von 14 Tagen. Die Plasmakonzentrationen bei den mit den verschiedenen Emulsionen behandelten Frühgeborenen wurden zu den einzelnen Messzeitpunkten (Tag 1, 3, 7, 14) einander gegenübergestellt (Fig 1 und 2). Dabei zeigte sich, dass nach 24 Stunden sowohl für Östradiol als auch für Progesteron signifikant höhere Plasmakonzentrationen mit der erfindungsgemäßen Emulsion erreicht wurden. Die im Median erreichten Plasmakonzentrationen von Östradiol an Tag 3, 7 und 14 lagen bei dieser *immer* über den Werten derer, die mit der Emulsion gemäß Stand der Technik erreicht wurden. Die untere Grenze der angestrebten Plasmakonzentrationen von Östradiol (2 000 pg/ml) und Progesteron (300 ng/ml) wurde mit der erfindungsgemäßen Emulsion in 91 % bzw. 46 % hingegen mit der Stand der Technik Emulsion nur in 64 % bzw. 43 % aller Fälle erreicht oder überschritten.

Bereits nach 24 Stunden waren die Plasmakonzentrationen beider Hormone bei der Substitution mit der erfindungsgemäßen Emulsion signifikant höher verglichen mit der Emulsion des Standes der Technik, was dem Ziel einer schnellstmöglichen Erreichung von im Mutterleib vorkommenden Plasmakonzentrationen entgegenkommt.

Zusammenfassend weisen die vorliegenden Daten auf eine bessere Verfügbarkeit von Östradiol und Progesteron gemessen an den erreichten Plasmakonzentrationen bei Frühgeborenen hin. Damit ist die erfindungsgemäße Emulsion zur Anwendung bei Frühgeborenen besser geeignet, als die vergleichbare Emulsion des Standes der Technik, die durch Mischen der Hormone mit der fertigen Emulsion hergestellt wurde.

## Patentansprüche

1. Verfahren zur Herstellung isotoner östrogen- und gestagenhaltiger Ölemulsionen zur intravenösen Verabreichung umfassend die Schritte
(A) Lösen der Hormone in der Ölphase und
(B) Emulgieren der Öl- in der Wasserphase
in Gegenwart eines Emulgators.

2. Hormonhaltige isotone Ölemulsion zur intravenösen Applikation umfassend Gestagen und Östrogen im Verhältnis von 2:1 bis 200:1, erhältlich durch ein Verfahren umfassend die Schritte
(A) Lösen der Hormone in der Ölphase und
(B) Emulgieren der Öl- in der Wasserphase
in Gegenwart eines Emulgators.

3. Ölemulsion nach Anspruch 2, **dadurch gekennzeichnet, dass** die Emulsion zwischen 1 und 60 Gew.-%, eines Östrogens und zwischen 1 und 50 Gew.-% eines Gestagens, bezogen auf die Gesamtzusammensetzung umfasst.

4. Ölemulsion nach irgendeinem der Ansprüche 2 bis 3, **dadurch gekennzeichnet, dass** das Östrogenen Östradiol und das Gestagen Progesteron ist.

5. Ölemulsion nach irgendeinem der Ansprüche 2 bis 4, **dadurch gekennzeichnet, dass** die Ölphase mittelkettigen Triglyceriden (MCT) mit einer Kettenlänge von 6 bis 12, vorzugsweise 8 bis 10 Kohlenstoffatomen, umfasst.

6. Ölemulsion gemäß irgendeinem der Ansprüche 2 bis 5, **dadurch gekennzeichnet, dass** sie bis zu 1,5 Gew.-% des Emulgators aufweist.

7. Verwendung der isotonen Ölemulsion gemäß irgendeinem der Ansprüche 2 bis 6 zur Herstellung eines Medikaments zur intravenösen Applikation von Östrogen und Gestagen zur postnatalen Hormonsubstitution von Frühgeborenen.

8. Verwendung der isotonen Ölemulsion gemäß irgendeinem der Ansprüche 2 bis 6 zur Herstellung eines Medikaments zur Behandlung neurologischer Schäden nach Schlaganfällen.
